# EUROPEAN PATENT APPLICATION

(11) **EP 1 782 794 A2**
(43) Date of publication of application: **09.05.2007**
(21) Application number: 06254712.0
(22) Date of filing: 11.09.2006
(51) Int. Cl.: A61K 9/00, A61K 9/02, A61K 47/12, A61K 47/14, A61K 47/32, A61P 15/02

(54) **Anhydrous composition containing an acid-acid buffer system**

(30) Priority: 12.09.2005 US 716232 P; 13.09.2005 US 716441 P
(71) Applicant: Mcneil-PPC, Inc, Skillman, NJ 08558 (US)
(72) Inventor: Ahmad, Nawaz, Monmouth Junction, NJ 08852 (US); Cui,Cheng-Ji, Pennington, NJ 08534 (US)
(74) Representative: Fisher, Adrian John

(57) **Abstract**

This invention relates to anhydrous compositions and methods of maintaining healthy vaginal pH to control vaginal odor. Such compositions may be applied to the vaginal area to lower vaginal pH and assist in maintaining such pH over a period of time.

## Description

### FIELD OF THE INVENTION

This invention relates to compositions and methods for treating vaginal conditions in which the pH is elevated beyond the normal, healthy range of 3.8 to 4.5. The composition may be useful for treating or ameliorating conditions which cause the pH to rise or to treat the presence of odor, which often results from this elevated pH. Additionally, this composition may further be used to maintain a healthy vaginal environment.

### BACKGROUND OF THE INVENTION

Normal vaginal pH ranges from 3.8 to 4.5. At this pH, the microbial flora which inhabit the healthy vagina, including lactobacilli, thrive. Furthermore, at this pH, opportunistic bacterial growth and attachment to the vaginal walls is inhibited. One important function that lactobacilli perform is modulating normal vaginal flora. They accomplish this both by producing lactic acid, which maintains an acidic pH and by producing hydrogen peroxide, which inhibits catalase-negative bacteria.

Certain activities and conditions can raise vaginal pH, including menstruation, intercourse and lack of estrogen, such as occurs in menopause. An elevated pH is also part of the differential diagnosis of bacterial vaginosis.

In women with bacterial vaginosis, there is a seven-fold decrease in the prevalence of facultative *Lactobacilli* and a corresponding increase in the population of other bacteria, some of which may be pathogenic. Symptomatic women with bacterial vaginosis have a 100-to 1000-fold increase in *Gardenerella vaginalis, Mobiluncus, Peptostreptococcus and Bacteroides.* Besides pruritus, yellow discharge and irritation, the most common symptom of vaginosis is odor, specifically an amine odor. The characteristic amine odor in bacterial vaginosis is created by products from anaerobic bacterial metabolism in the vaginal fluid of women with bacterial vaginosis. The elevation of pH volatizes amines from protein attachment and produces the odor.

It is therefore may be desirable for individuals to restore "healthy" vaginal pH whenever an increase in the vaginal pH is observed. Not only would this restoration appear to support or promote the maintenance of a healthy selection of vaginal flora, it may assist in controlling odor that may be caused by the growth of pathogenic bacteria.

U.S. Patent Number 6,017,521 (Robinson et al.) describes the use of water-swellable, water-insoluble carboxylic acid polymers in aqueous compositions that may be administered to the vagina to lower pH of the vagina. Carboxylic acid polymers such as carbopols and carbomers, are high molecular weight, cross-linked, acrylic acid-based polymers. Carbopols have been extensively used as gelling and viscosity building agents in pharmaceutical and cosmetic applications. The pH of a 1% water dispersion of these Carbopol polymers is generally between about 2.5 and about 3. The molecules of these acrylic acid polymers are highly coiled in conformation, thus limiting their solubility and thickening capability. The polymers described in Robinson, et al., polycarbophils, tend to be water insoluble and water swellable. When dispersed in water, the molecule begins to hydrate and uncoil slightly, generating an increase in viscosity. In order to achieve the highest possible performance with the polymer, the molecule should be completely uncoiled. As the molecule uncoils, its solubility increases. Generally, neutralizing the polymer with a suitable base like sodium or potassium hydroxide is a method used to uncoil these molecules, a rapid reaction that instantaneously thickens the composition in which the polymer resides. Although Carbopol polymers can be used without neutralization, neutralization aids in solubility.

However, Robinson et al. does not provide an anhydrous composition that allows the polymer to become neutralized when exposed to a relatively basic environment.

Organic acids such as acetic, sorbic, lactic, citric, tartaric acid and the like used alone and applied to the vagina will lower the pH but will not maintain a low pH because of they have a low buffering capacity.

Thus, an anhydrous composition which would lower pH and maintain a lower pH in the vagina is desirable.

### SUMMARY OF THE INVENTION

This invention relates to anhydrous compositions containing an "acid-acid buffer system" that are intended to provide adjustment and maintenance of an acidic vaginal pH due to their high buffering capacity *in vitro.* Preferably, the pH to which the vagina is adjusted is between about 2.5 and about 5, more preferably. lower than 4.7. The "acid-acid buffer system" of this invention contains both an acidic polymer and an organic acid. Unexpectedly, the combination of such acidic polymers and organic acids in an anhydrous formulation produces compositions having a surprisingly high buffer capacity. As used herein, the term "anhydrous" means a composition containing less than about 20% by weight of water, more preferably, less than about 10% by weight of water and most preferably, less than about 5% by weight of water.

In accordance with this invention, we believe that the compositions of this invention come into contact with the aqueous contents and lining of the vagina and its components dissolve to generate an acidic pH between about 2.5 and about 5. The resulting acid-acid based buffer has a very high buffering capacity and it is believed that it will resist the changes in the pH by dilution or other changes affecting vaginal environment over its residence time in the vaginal vault. This high buffering capacity buffer has been observed to be capable of accepting up to 24 times its volume of pH 7 buffer *in vitro* and still maintain a pH below about 5. This should potentially enable the vagina to maintain a healthy vaginal flora with a reduced vaginosis causing organisms that will result in elimination or reduction in vaginal odor.

The compositions of this invention preferably contain water-soluble acrylic acid polymers in their non-neutralized acidic state alone or in combination with organic acids such as alphahydroxy acids, more particularly lactic acid, acetic acid, tartaric acid and the like, in a suitable concentration to deliver a buffer with a high buffering capacity. This generates a unique acid-acid buffer that not only delivers a lower overall pH but also posses a much higher buffering capacity than seen any the prior art.

Water-soluble polymers with ionizable functional groups, including, for example, a carboxylic acid and/or an amine group, and a buffering capacity may also be used as polymeric buffers according to this invention. Preferably, water-soluble polymers having acrylic acid groups may be used in the compositions of this invention.

We believe that, in their non-neutralized state, such water-soluble acrylic acid polymers, when they come into contact with the higher vaginal pH of the women with bacterial vaginosis, undergo neutralization and uncoiling and will thereby create a high viscosity bioadhesive gel.

### BRIEF DESCRIPTION OF THE DRAWINGS

FIG. 1 is a graph comparing the buffering capacity of 4% Carbopol 974P in anhydrous base (Composition A), 3% lactic acid solution in the anhydrous base (Composition B) and 4% Carbopol and 3% lactic acid in the anhydrous base with RepHresh^{®} Vaginal Gel .
FIG. 2 is a graph comparing the buffering capacity of composition Example 1 and Example 2 of the invention with the RepHresh^{®} Vaginal Gel.
FIG. 3 is a graph comparing the buffering capacity of composition Example 3 and Example 4 of the invention with the RepHresh^{®} Vaginal Gel.
FIG. 4 is a graph comparing the *in vivo* pH effects of applying compositions of this invention, a composition as set forth in copending patent application Serial No. (Attorney Docket No. PPC833CIP2 filed concurrently herewith) and RepHresh^{®} Vaginal Gel.

### DETAILED DESCRIPTION OF THE INVENTION

### Compositions Of the Invention

The compositions of this invention relate to anhydrous buffering systems containing water-soluble polyacrylic polymers in combination with one or more organic acids. The compositions of this invention also relate to anhydrous buffering systems in which water-soluble polyacrylic polymers can be used alone in unique concentrations to create low pH buffers with high buffering capacity. Preferably, these polymers should be present in the amount of at least about 2% by weight, more preferably, at least about 3% by weight.

The methods of this invention relate to the use of the compositions of this invention for lowering and maintaining vaginal pH. We believe that these compositions will adjust and maintain an acidic pH in the vagina, thereby supporting the inhibition and control of the growth of microorganisms causing bacterial vaginosis and vaginal odor.

Although the water-soluble polyacrylic polymers disclosed in these compositions have been previously known and used, we believe that these uses have been limited to employment in aqueous compositions as consistency enhancing agents. Similarly, the organic acids used in these compositions have been used as acidifying or pH adjusting agents; these have not, to our knowledge, been utilized in anhydrous compositions.

The buffering compositions of this invention are designed for vaginal use. The water-soluble polyacrylic polymers of this invention, in an anhydrous non-neutralized state remain uncoiled and retain their bioadhesive potential to maximum capacity. When exposed to the comparatively higher vaginal pH of the patients suffering from vaginosis, these polymers undergo uncoiling and become bioadhesive, thus adhering to the vaginal epithelium and prolonging the duration of action, and thereby maintaining a low healthy vaginal pH for a long period of time. Because the polyacrylic acids do not hydrate and build viscosity in the anhydrous compositions of this invention, they can be used at substantially higher concentration as compared with their aqueous use. When delivered at substantially higher concentrations in the anhydrous compositions of this invention, these polymers are expected to achieve a surprisingly high viscosity and bioadhesion.

The compositions of this invention preferably contain polyacrylic polymers that are cross-linked with allyl sucrose or allylpentaerythritol, which makes them water-soluble. Examples of such polymers are Carbopol^{®} polymers, available from NOVEON, B.F. Goodrich Chemical Corp. of Cleveland, Ohio. Aqueous dispersions of these polymers have an approximate pH range of 2.8 to 3.2 depending upon the polymer concentration. In the anhydrous compositions of this invention, the polyacrylic polymers will act as acidic buffers alone or in combination with organic acids when they come in contact with the vaginal moisture. Examples of the polyacrylic acid polymers most preferably used in the current invention are sold under the trade name Carbopol^{®}, and include Carbopol^{®} 974, Carbopol^{®} 934 and, Carbopol^{®} 980 all by NOVEON, B.F.Goodrich Chemical Corp of Cleveland Ohio. Preferably, the acidic polymers are present in the compositions of this invention in the amount of from about at least about 2% by weight, more preferably, at least about 3% by weight of the composition.

### Buffering Capacity

Standard classical buffers known to those of ordinary skill in the art generally contain an acid in combination with a base to maintain a certain pH within a composition. Buffers developed in this manner generally resist changes in the pH and maintain the original pH of the buffer. The extent to which these buffers can resist change in pH is designated as their "buffering capacity". The anhydrous acid-acid buffers of this invention are surprisingly different from the standard buffers known to those of ordinary skill in the art in that they have a substantially high buffering capacity than known classical buffer systems.

The classical method of determination of buffering capacity of a buffer is to titrate the buffer using 1 Normal Sodium Hydroxide Solution and mg of NaOH is calculated per 5g of the test sample. Because the acidic compositions of this invention will not be subjected to an interaction with sodium hydroxide normal human use, we have modified the method for determining buffering capacity in accordance with simulated human conditions. Instead of 1 N sodium hydroxide solution, a standard pH 7 buffer was used for titration. The pH of a 10 g sample of the test article was measured and, to this sample, pH 7 buffer was added in 5 ml increments with continuous mixing while monitoring the resulting pH change. The titration was discontinued when pH reached around 7.

### Buffering Systems of the Invention

The compositions of this invention relate to acid-acid buffering systems containing water soluble acrylic acid polymers known to the art as Carbopols^{®}, alone or in combination with organic acids known to the art such as alphahydroxy acids, more preferably benzoic acid, alginic acid, sorbic acid, stearic acid, oleic acid, edetic acid, gluconodeltalactone, acetic acid, fumaric acid, lactic acid, citric acid, propionic acid, malic acid, succinic acid, gluconic acid, ascorbic acid and tartaric acid and the like. Preferably, the compositions of this invention contain organic acid in the amount of from about at least about 2% by weight of the composition, more preferably at least about 3% by weight.

Typical buffer systems of this invention are summarized in Table 1 as examples. The concentration of polyacrylic polymer and organic acid in these buffer systems can be customized in order to deliver a desired pH and buffering capacity, depending on other ingredients and combination of ingredients in the compositions set forth herein.

**Table 1**

| **Buffers of The Invention** | | | |
|---|---|---|---|
| **INGREDIENT** | **SOLUTION A Polyacrylic Polymer Alone** | **SOLUTION B Lactic Acid Alone** | **Solution C Polyacrylic Polymer-Lactic Acid Combination** |
| INGREDIENT | % w/w | % w/w | % w/w |
| Carbopol 974P | 4.00 | | 4.00 |
| Propylene Glycol | 72.00 | 72.75 | 69.75 |
| PEG 400 | 24.00 | 24.25 | 23.25 |
| Lactic Acid | | 3.00 | 3.00 |
| Total | 100.00 | 100.00 | 100.00 |

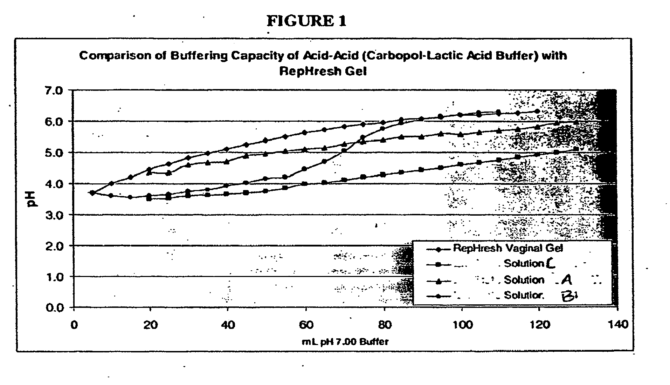

The anhydrous buffers of this invention can accept many times their own weight of pH 7 buffer solution and can be measured to be preferably below 5, even after the addition of 24 times its weight of pH 7 buffer. As set forth in Figure 1, the pH of RepHresh^{®} Vaginal Gel after addition of the corresponding quantity of pH 7 buffer is 6.32, a difference of 1.70 pH units. (Fig.1, Buffer Solution C).

The compositions of this invention are intended for vaginal application and use. The preferred compositions of this invention may be made as anhydrous gels, films, vaginal suppositories or inserts, ointments, gelatin capsules, polymeric suppositories, vaginal washes and other dosage forms suitable for vaginal use.

The compositions of this invention may contain additional components in conjunction with the buffer systems of this invention. The relative quantities of these components may vary according to the desired nature and consistency of the intended dosage form or application. These components include polyols, such as polyhydric alcohols, including but not limited to the following: glycerin, propylene glycol, polyethylene glycol 1000, polyethylene glycol 400, polyethylene glycol 145 and polyethylene glycol 400. The buffering compositions of this invention may also contain polyols in accordance with relative quantities that vary according to the desired nature and consistency of the intended dosage form or application. Preferably, the compositions of this invention contain at least one polyol. Preferably, the polyol is a polyhydric alcohol, and more preferably, at least two polyhydric alcohols. Polyethylene glycol (hereinafter, "PEG") ethers may also be used, including PEG ethers of propylene glycol, propylene glycol stearate, propylene glycol oleate and propylene glycol cocoate and the like. Specific examples of such PEG ethers include PEG-25 propylene glycol stearate, PEG-55 propylene glycol oleate and the like. Preferably, at least one of the polyhydric alcohols of the compositions of this invention is a polyalkylene glycols or others selected from the following group: glycerine, propylene glycol, butylene glycol, hexalene glycol or polyethylene glycol of various molecular weight and the like and/or combination thereof. More preferably, the compositions of this invention contain a polyethylene glycol; most preferably, the polyethylene glycol may be selected from the following group: polyethylene glycol 400 or polyethylene glycol 300. Polypropylene glycol of various molecular weights may also be used. PEGylated compounds such as peptide or protein derivatives obtained through PEGylation reactions may also be used. In addition, block copolymers of PEG's may be used, such as (ethylene glycol)-block-poly(propylene glycol)-block-(polyethylene glycol), poly(ethylene glycol-ran-propylene glycol) and the like. These polyols may be useful in the compositions of this invention as solvents, humectants or carriers.

The compositions may also include fatty alcohols such stearyl alcohol, cetyl alcohol, which act as emollients and enhancing agents. The compositions may also include hydrogenated vegetable oils as suppository bases. The compositions may also include cellulosic polymers as viscosity building and enhancing agents and may include but are not limited to hydroxyalkylcelluloses, including hydroxymethylcellulose, hydroxyethycellulose, hydroxypropycellulose and hydropropylmethylcellulose.

Other components may be present in the compositions of this invention such as anti-oxidants, chelating agents, preservatives, oils, waxes, surfactants, viscosity building agents, solvents, moisturizing agents, solubilizers and bioadhesives/mucoadhesives and the like. The relative quantities of such components may vary according to the desired nature and consistency of the composition, including creams, ointments, waxy suppositories, gelatin capsules, anhydrous polymeric suppositories and the like.
The anhydrous buffering systems according to this invention may be applied into the vagina on a regular basis to maintain vaginal pH. They may also be utilized as an adjunct to, for example, prior to, during, or after an intravaginal antifungal or antibacterial drug treatment. The buffering systems may be co-administered with the antifungal azole in the same composition, or as two different or separate compositions, but administered together or substantially simultaneously. For example, the buffering systems may be incorporated directly into a composition containing an antifungal azole compound. In this case, the buffering systems and the azole compound are preferably administered to patients simultaneously during application. The buffering systems may be coated on the outer surface of an vaginal suppository (e.g., a wax- or fatty acid based antifungal or antibacterial vaginal suppository), or a gelatin capsule suppository containing an antifungal drug. The buffering systems may also be incorporated into the gelatin-wall of the antifungal or antibacterial gelatin capsule.

Alternatively, the respective antifungal or antibacterial composition and buffering composition may be administered sequentially and separated by a certain time period. For example, a composition for intravaginal application may contain only the buffering system without any antifungal azole. Such buffering composition may be administered into the vagina when an antifungal or antibacterial composition is present in the vagina. It is believed that the active antifungal or antibacterial compound already in the vagina resulted from an earlier intravaginal or oral antifungal or antibacterial treatment, and the buffering systems work to extend the intended therapeutic efficacy to treat or prevent the occurrence of bacterial vaginosis. Because the active compound in vaginal tissue and fluid usually lasts for several days following an intravaginal administration, the buffer system may be administered preferably from less than one hour to about 10 days, more preferably, from about 8 hours to about 7 days, and most preferably, from about 12 hours to about 5 days, after administration of an active ingredient.

The compositions of this invention are illustrated, but should not considered to be limited, by the following Examples.

### Examples 1-10

The compositions of Examples 1, 2, 5, 7, 9 and 10 of this invention may be made as follows: first, the carbopol and acid are mixed propylene glycol and glycerin are mixed. A polyacrylic polymer, organic acid and a fatty acid (if present) are then added to the mixture in the same container. The mixture is then heated to from about 35°C to about 45°C to homogenize the mixture. The mixture is then cooled.

The compositions of Examples 3, 4, 6 and 8 of this invention (anhydrous suppositories) may be made as follows: the suppository base, such as polyethylene glycols and/or hydrogenated vegetable oils, is melted by heating at about 60°C. The acidic polyacrylic polymer and organic acid are added with mixing. Mixing is continued for 30 minutes or until the mixture is homogeneous. The mixture is cooled and maintained at a temperature of about 40°C to about 45°C and formed into suppositories. Preferably, they may also be made by adding an organic acid to the polyacrylic polymer and heating to about 35° to about 50°C while mixing the formulation. The other components are then added to this mixture and the entire formulation stirred until it is homogeneous. The suppositories are permitted to cool and dry.

### Example 1 (Anhydrous Gel)

| Ingredient | % w/w |
|---|---|
| Polyethylene Glycol 1000 | 89.30 |
| Carbopol 974P | 4.00 |
| Lactic Acid | 2.00 |
| Stearyl Alcohol | 4.70 |
| Total | 100.00 |

### Example 2 (Anhydrous Gel)

| Ingredient | % w/w |
|---|---|
| Polyethylene Glycol 400 | 63.00 |
| Carbopol 974P | 5.00 |
| Lactic Acid | 3.00 |
| Stearyl Alcohol | 4.00 |
| Total | 100.00 |

### Example 3 (Anhydrous Suppository)

| Ingredient | % w/w |
|---|---|
| Polyethylene Glycol 1000 | 96.00 |
| Carbopol 974P | 3.00 |
| Lactic Acid | 1.00 |
| Total | 100.00 |

### Example 4 (Anhydrous suppository)

| Ingredient | % w/w |
|---|---|
| Polyethylene Glycol 1000 | 47.00 |
| Carbopol 974P | 4.00 |
| Lactic Acid | 2.00 |
| Hydrogenated Vegetable Oil | 47.00 |
| Total | 100.00 |

### Example 5 (Anhydrous Gel)

| Ingredient | % w/w |
|---|---|
| Polyethylene Glycol 400 | 23.00 |
| Propylene Glycol | 69.00 |
| Carbopol 974P | 2.00 |
| Lactic Acid | 3.00 |
| Stearyl Alcohol | 3.00 |
| Total | 100.00 |

### Example 6 (Anhydrous Suppository)

| Ingredient | % w/w |
|---|---|
| Polyethylene Glycol 1000 | 83.00 |
| Polyethylene Glycol 400 | 10.00 |
| Carbopol 974P | 4.00 |
| Lactic Acid | 3.00 |
| Total | 100.00 |

### Example 7 (Anhydrous Gel)

| Ingredient | % w/w |
|---|---|
| Polyethylene Glycol 400 | 21.00 |
| Propylene Glycol | 66.00 |
| Carbopol 974P | 5.00 |
| Lactic Acid | 5.00 |
| Stearyl Alcohol | 3.00 |
| Total | 100.00 |

### Example 8 (Anhydrous Suppository)

| Ingredient | % w/w |
|---|---|
| Polyethylene Glycol 1000 | 80.00 |
| Polyethylene Glycol 400 | 10.00 |
| Carbopol 974P | 5.00 |
| Lactic Acid | 5.00 |
| Total | 100.00 |

### Example 9 (Anhydrous Gel)

| Ingredient | % w/w |
|---|---|
| Polyethylene Glycol 1000 | 79.60 |
| Polyethylene Glycol 400 | 10.00 |
| Carbopol 974P | 5.00 |
| Lactic Acid | 5.00 |
| Hydroxypropylcellulose | 0.40 |
| Total | 100.00 |

### Example 10 (Anhydrous Gel)

| Ingredient | % w/w |
|---|---|
| Polyethylene Glycol 1000 | 78.75 |
| Polyethylene Glycol 400 | 10.00 |
| Carbopol 974P | 5.00 |
| Lactic Acid | 5.00 |
| Hydroxypropylcellulose | 1.25 |
| Total | 100.00 |

**Table 2**

| **Buffering Capacity of The Buffer of the Invention** | | |
|---|---|---|
| **ml of pH 7 Buffer Used** | **RepHresh® Vaginal Gel** | **Composition C of the Invention** |
| 20 | 4.44 | 3.50 |
| 40 | 5.09 | 3.65 |
| 60 | 5.62 | 3.97 |
| 80 | 5.96 | 4.27 |
| 100 | 6.19 | 4.59 |
| 120 | 6.32 | 4.92 |

**Table 3**

| **Buffering Capacity of Compositions of The Invention** | | | | | |
|---|---|---|---|---|---|
| **ml of pH 7 Buffer Used** | **pH** | | | | |
| | **RepHresh Vaginal Gel** | **Example 1 of the Invention** | **Example 2 of the Invention** | **Example 3 of the Invention** | **Example 4 of the Invention** |
| 20 | 4.44 | 4.02 | 3.84 | 4.39 | 3.91 |
| 40 | 5.09 | 4.15 | 3.19 | 4.37 | 3.98 |
| 60 | 5.62 | 4.42 | 4.14 | 4.66 | 4.20 |
| 80 | 5.96 | 4.82 | 4.35 | 5.01 | 4.45 |
| 100 | 6.19 | 5.15 | 4.59 | 5.37 | 4.80 |
| 120 | 6.32 | 5.29 | 4.64 | 5.68 | 5.11 |

### Example 11: pH and Buffering Capacity

The pH and buffering capacity of Composition C of this invention and that of RepHresh^{®} Vaginal Gel, a commercially-available product from Columbia Laboratories, Inc. of Livingston, NJ, are summarized in Table 2 and graphically illustrated in Figure 1. As illustrated by the data set forth in Table 3, after the addition of 120 ml of pH 7 buffer, the pH of the buffer of the composition of this invention is below pH 5, 4.92 to be exact while that of the RepHresh^{®} Vaginal Gel is higher than pH 6 (6.32).

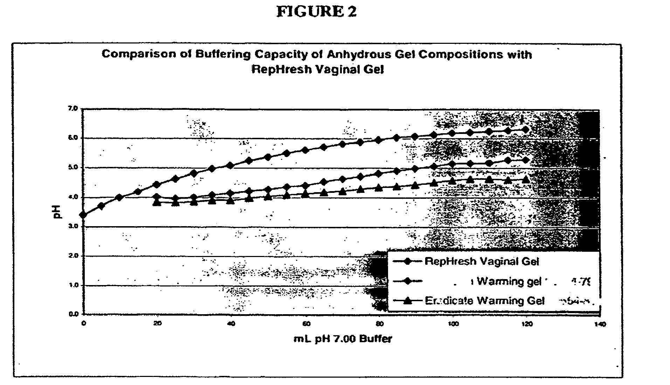

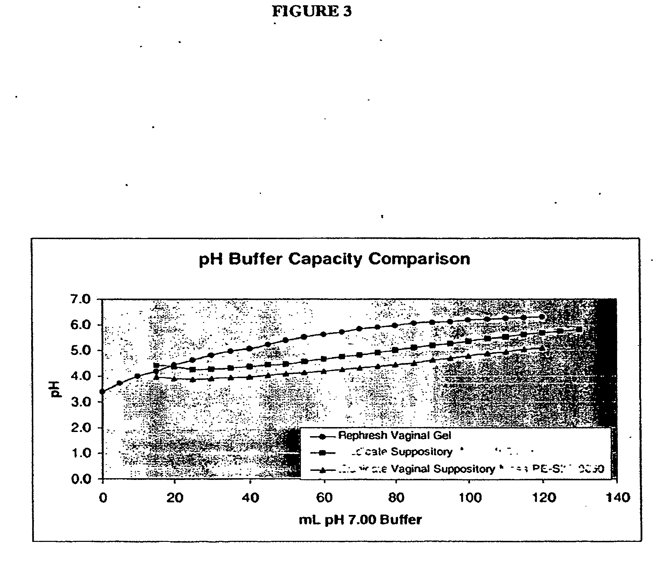

The pH and the buffering capacities of representative compositions of this invention are illustrated in Figure 2 and Figure 3. These are typical Buffering Capacity plots for the two anhydrous gels, Example 1 (anhydrous gel) and Example 2 (anhydrous gel) and two vaginal suppositories, Example 3 (suppository) and Example 4 (suppository) of the current invention. This data is also summarized in Table 2. pH 7 buffer was added in increments of 5 ml to a 10 g sample of the product and pH of the resulting combination was determined. The pH data illustrates that a much lower pH was consistently maintained by the compositions of this invention throughout the successive additions of pH 7 buffer as compared with RepHresh^{®} Vaginal Gel. Surprisingly, the compositions of this invention have much lower pH profile and a much higher buffering capacity. In actual human use, we expect that the compositions of the invention would be able to maintain a healthy vaginal pH.

### Example 12: Bacterial Vaginosis and Time Kill Test

### In Vitro Testing for Antibacterial Activity

*In vitro* Time-Kill Studies were performed in order to test the antibacterial activity of the compositions of this invention against bacterial vaginosis-causing organisms, namely, *Gardnerella, Mobilincus* and *Peptostrep.* A battery of vaginal anaerobes known to cause bacterial vaginal infections (BV), and strains of *lactobacilli* were used to determine the length of contact time required to inhibit and kill these test organisms. The results of this test are summarized in Table 3. The results show that Compositions 1, and 4 of the invention kill the BV causing bacteria in 0 hour or almost instantaneously.

These results show that the gels and suppositories of this invention will be effective to treat bacterial vaginal infections or BV. They will be able to minimize or eliminate vaginal odor by treating vaginosis.

**Table 4**

| **Results of *In Vitro* Evaluation: Activities of Compositions of the Invention and That of RepHresh^{®} Vaginal Gel Against BV Organisms** | | | |
|---|---|---|---|
| **Compound:** | **Anhydrous Gel Example 1** | **Suppository Example 4** | **RepHresh Gel** |
| **Organism:** | | | |
| Gardnerella | | | |
| vaginalis | 0 | <1 | <1 |
| G. vaginalis | 0 | <1 | <1 |
| G. vaginalis | <1 | <1 | <1 |
| G. vaginalis | <1 | <1 | 1 |
| Peptostrep. magnus | 1 | 1 | 2 |
| Ps. Magnus | <1 | 1 | 3 |
| Ps. Anaerobius | 0 | <1 | <1 |
| Ps. Anaerobius | <1 | <1 | 1 |
| Ps. Tetradius | 0 | 0 | <1 |
| Ps. Tetradius | <1 | <1 | 1 |
| Ps. lactolyticus | <1 | <1 | 1 |
| Ps. lactolyticus | <1 | <1 | 1 |
| Ps. | | | |
| asaccharolyticus | <1 | <1 | <1 |
| Ps. | | | |
| asaccharolyticus | <1 | 1 | 1 |
| Prevotella bivia | <1 | <1 | <1 |
| Prev. bivia | <1 | <1 | <1 |
| Prev. disiens | 0 | <1 | <1 |
| Prev. disiens | 0 | 0 | 0 |
| Prev. intermedia | 0 | 0 | 0 |
| Prev. intermedia | 0 | 0 | 0 |
| Prev. | | | |
| melaninogenica | <1 | <1 | 1 |
| Prev. | | | |
| melaninogenica | <1 | 0 | 0 |
| Lacto. Jensenii | 1 | 6 | >8<24 |
| Lacto. Jensenii | 2 | >8<24 | >8<24 |
| Lacto. Gasseri | 7 | >24 | >24 |
| Lacto. Iners | <1 | 1 | <1 |
| Mobil. Mulieris | <1 | <1 | 1 |
| Mobil. Mulieris | <1 | <1 | 1 |
| Mobil. Curtisii | <1 | 1 | 2 |
| Mobil. Curtisii | <1 | 1 | 2 |
| B. fragilis | 2 | >8<24 | >8<24 |
| B. thetaiotaomicron | 1 | >8<24 | >8<24 |

### Example 13: Clinical Study to Determine Vaginal pH and Perceived Vaginal Odor Control

A clinical study was performed to determine whether the compositions of this invention were capable of controlling or affecting vaginal pH and perceived vaginal odor in human subjects. One hundred and twenty-five women who completed the study were provided with one of the products set forth in Table 5 to apply vaginally. Composition A is an aqueous gel as set forth in copending patent application Serial No. 11/224,189 (Attorney Docket No. PPC833CIP2 filed concurrently herewith); Composition B is an anhydrous gel as set forth in Example 1 above; Composition C is an anhydrous suppository as set forth in Example 6 above; Composition D is a sample of RepHresh Vaginal Gel as described in U.S. Patent No. 6,017,521 and commercially available from Columbia Laboratories, Inc. of Livingston, NJ.

The subjects' vaginal pH was tested by vaginal examination with a pH indicator stick and pH electrode probe at baseline and at approximately 1, 6, 24, 48, 72 and 96 hours following the test article application. The women were also asked to assess their self-perceived level of vaginal odor. The results of this test are set forth in Tables 6 and 7 below. Table 6 shows that Compositions B and C of this invention effected a significant decrease in vaginal pH score compared with baseline. Table 7 shows that the use of Compositions B and C of this invention resulted in significant decrease of self-perceived odor compared with baseline.

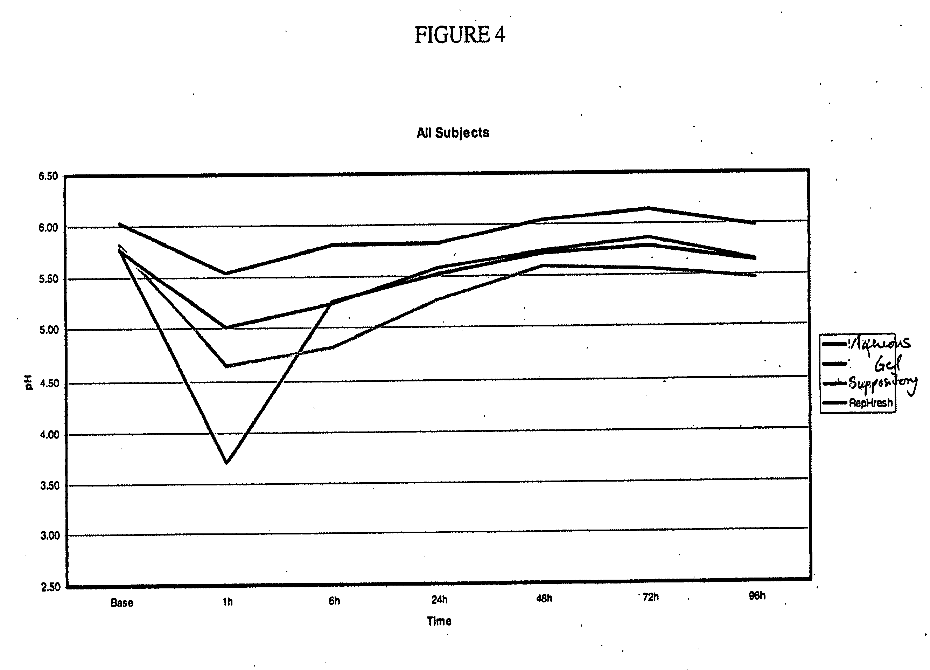

Another, preliminary clinical study was performed earlier than that set forth above in a significantly smaller group with the same formulations. Their use resulted in an immediately lower vaginal pH and lowered the self-perceived vaginal odor of the subjects. The plot of measured pH over time is set forth in Figure 4.

**Table 6:**

| **Vaginal pH Levels using the pH Electrode Probe Within-Treatment Analysis** | | | | | | | |
|---|---|---|---|---|---|---|---|
| **HTR Code** | **Visit** | **Mean Score (std. dev.)** | | **Mean Change from Baseline (std. dev.)** | | **n** | **t-test p-value** |
| | Baseline | 5.75 | (0.83) | --- | | 30 | **---** |
| | 1 Hour | 3.58 | (0.72) | -2.17 | (0.92) | 30 | **<0.0001¹** |
| | 6 Hours | 5.18 | (1.12) | -0.57 | (0.93) | 30 | **0.0022¹** |
| **A** | Day 2 | 5.46 | (0.96) | -0.29 | (0.75) | 30 | **0.0435¹** |
| | Day 3 | 5.67 | (0.94) | -0.08 | (0.78) | 30 | >0.5000 |
| | Day 4 | 5.70 | (1.19) | -0.05 | (0.92) | 30 | >0.5000 |
| | Day 5 | 5.64 | (1.00) | -0.11 | (0.66) | 30 | 0.3741 |
| | Baseline | 6.02 | (1.01) | --- | | 23 | --- |
| | 1 Hour | 4.94 | (1.45) | -1.08 | (0.66) | 23 | **<0.0001¹** |
| | 6 Hours | 5.56 | (1.10) | -0.48 | (0.65) | 21 | **0.0030¹** |
| **B** | Day 2 | 5.41 | (1.10) | -0.60 | (0.56) | 22 | **0.0001¹** |
| | Day 3 | 5.76 | (1.31) | -0.34 | (0.54) | 20 | **0.0100¹** |
| | Day 4 | 5.83 | (1.15) | -0.28 | (0.73) | 20 | 0.1078 |
| | Day 5 | 5.84 | (1.49) | -0.26 | (0.75) | 20 | 0.1378 |
| | Baseline | 5.80 | (0.83) | --- | | 28 | --- |
| | 1 Hour | 4.48 | (0.77) | -1.32 | (0.93) | 28 | **<0.0001¹** |
| | 6 Hours | 4.65 | (0.85) | -1.17 | (0.91) | 27 | **<0.0001¹** |
| **C** | Day 2 | 5.15 | (0.83) | -0.66 | (0.88) | 28 | **0.0005¹** |
| | Day 3 | 5.51 | (1.05) | -0.29 | (0.82) | 27 | 0.0772 |
| | Day 4 | 5.37 | (1.08) | -0.44 | (0.84) | 28 | **0.0108¹** |
| | Day 5 | 5.28 | (1.01) | -0.50 | (0.80) | 26 | **0.0038¹** |
| | Baseline | 5.84 | (0.69) | --- | | 25 | --- |
| | 1 Hour | 4.76 | (0.69) | -1.08 | (0.67) | 25 | **<0.0001¹** |
| | 6 Hours | 5.06 | (0.85) | -0.78 | (0.94) | 25 | **0.0003¹** |
| **D** | Day 2 | 5.49 | (1.07) | -0.35 | (0.93) | 25 | 0.0760 |
| | Day 3 | 5.73 | (1.00) | -0.11 | (0.71) | 24 | 0.4581 |
| | Day 4 | 5.71 | (1.00) | -0.12 | (0.72) | 25 | 0.4003 |
| | Day 5 | 5.52 | (1.02) | -0.31 | (0.91) | 25 | 0.0979 |

| | | | | | | | |
|---|---|---|---|---|---|---|---|
| ¹ Significant decrease in score compared to baseline. | | | | | | | |

**Table 7**

| **Self-Perceived Vaginal Odor Within-Treatment Analysis** | | | | | | | |
|---|---|---|---|---|---|---|---|
| **HTR Code** | **Visit** | **Mean Score (std. Dev.)** | | **Mean Change from Baseline (std. dev.)** | | **n** | **t-test p-value** |
| | Baseline | 2.77 | (0.82) | --- | | 30 | **---** |
| | 1 Hour | 2.10 | (1.01) | -0.62 | (0.86) | 29 | **0.0006¹** |
| | 6 Hours | 1.96 | (0.84) | -0.79 | (0.96) | 28 | **0.0002¹** |
| **A** | Day 2 | 1.73 | (0.78) | -1.03 | (0.67) | 30 | **<0.0001¹** |
| | Day 3 | 1.93 | (0.87) | -0.83 | (0.87) | 30 | **<0.0001¹** |
| | Day 4 | 1.93 | (0.83) | -0.83 | (0.83) | 30 | **<0.0001¹** |
| | Day 5 | 1.93 | (0.94) | -0.83 | (0.83) | 30 | **<0.0001¹** |
| | Baseline | 2.83 | (1.07) | --- | | 23 | --- |
| | 1 Hour | 2.09 | (0.92) | -0.73 | (0.94) | 22 | **0.0015¹** |
| | 6 Hours | 2.20 | (0.95) | -0.65 | (0.81) | 20 | **0.0020¹** |
| **B** | Day 2 | 2.14 | (1.06) | -0.71 | (0.78) | 21 | **0.0005¹** |
| | Day 3 | 2.24 | (0.77) | -0.62 | (0.92) | 21 | **0.0059¹** |
| | Day 4 | 2.19 | (0.87) | -0.67 | (0.97) | 21 | **0.0049¹** |
| | Day 5 | 2.14 | (0.79) | -0.71 | (0.90) | 21 | **0.0017¹** |
| | Baseline | 2.96 | (0.81) | --- | | 27 | --- |
| | 1 Hour | 2.29 | (1.01) | -0.63 | (0.97) | 27 | **0.0023¹** |
| | 6 Hours | 2.08 | (1.00) | -0.84 | (0.94) | 25 | **0.0002¹** |
| **C** | Day 2 | 2.00 | (0.82) | -0.96 | (1.02) | 27 | **<0.0001¹** |
| | Day 3 | 1.82 | (0.86) | -1.15 | (1.03) | 27 | **<0.0001¹** |
| | Day 4 | 1.68 | (0.94) | -1.30 | (1.07) | 27 | **<0.0001¹** |
| | Day 5 | 1.75 | (0.97) | -1.22 | (1.01) | 27 | **<0.0001¹** |
| | Baseline | 2.72 | (0.89) | --- | | 25 | **---** |
| | 1 Hour | 2.28 | (0.94) | -0.44 | (1.00) | 25 | **0.0383¹** |
| | 6 Hours | 2.21 | (0.88) | -0.54 | (0.93) | 24 | **0.0091¹** |
| **D** | Day 2 | 2.12 | (0.97) | -0.60 | (1.29) | 25 | **0.0289¹** |
| | Day 3 | 1.88 | (0.78) | -0.84 | (1.03) | 25 | **0.0004¹** |
| | Day 4 | 1.88 | (1.01) | -0.84 | (1.28) | 25 | **0.0032¹** |
| | Day 5 | 1.80 | (0.96) | -0.92 | (1.32) | 25 | **0.0019**¹ |

| | | | | | | | |
|---|---|---|---|---|---|---|---|
| Scale: 1=None, 2=slight, 3=moderate, 4=strong, 5=very strong ¹ Significant decrease in score compared to baseline. | | | | | | | |

## Claims

1. A composition comprising an acid-acid buffer system, wherein the composition is anhydrous.

2. A composition according to claim 1, wherein said acid-acid buffer system comprises a water-soluble acidic polymer and an organic acid.

3. A composition according to claim 2, wherein the acidic polymer is a polyacrylic polymer.

4. A composition according to claim 3, wherein the polyacrylic polymer is crosslinked with allyl sucrose or allylpentaerythritol.

5. A composition according to claim 4 wherein said polyacrylic polymer is a carbomer.

6. A composition according to claim 2, wherein the organic acid is an alphahydroxy acid.

7. A composition according to claim 2 wherein said organic acid is selected from the group consisting of benzoic acid, alginic acid, sorbic acid, stearic acid, oleic acid, edetic acid, gluconodeltalactone, acetic acid, fumaric acid, lactic acid, citric acid, propionic acid, malic acid, succinic acid, gluconic acid, ascorbic acid and tartaric acid.

8. A composition according to claim 1 or claim 2, further comprising propylene glycol.

9. A composition according to claim 1 or claim 2, further comprising polyethylene glycol.

10. A composition according to claim 1 wherein said composition is in the form of a vaginal suppository.

11. A composition according to claim 10 wherein said composition further comprises hydrogenated vegetable oil.

12. Use of a composition according to any one of claims 1 to 11 for lowering vaginal pH.

13. Use of a composition according to any one of claims 1 to 11 for reducing self-perceived vaginal odor.
